# EUROPÄISCHE PATENTSCHRIFT

(11) **EP 2 486 900 B1**
(45) Veröffentlichungstag und Bekanntmachung des Hinweises auf die Patenterteilung: **29.10.2014**
(21) Anmeldenummer: 12154876.2
(22) Anmeldetag: 10.02.2012
(51) Int. Cl.: A61F 5/455

(54) **Harnableitvorrichtung für weibliche Personen**
Urine direction device for female persons
Dispositif d'évacuation d'urée pour sujets féminins

(30) Priorität: 14.02.2011 DE 202011002726 U
(43) Veröffentlichungstag der Anmeldung: 15.08.2012
(73) Patentinhaber: Jaeniche, Wilhelm, 77694 Kehl-Leutesheim (DE)
(72) Erfinder: Grundke, Reinhold, 84547 Emmerting (DE)
(74) Vertreter: Hofstetter, Schurack & Partner

(56) Entgegenhaltungen:
- DE-A1- 2 447 642
- US-A- 4 194 508
- US-A- 5 045 078

## Beschreibung

Die vorliegende Erfindung betrifft eine Harnableitvorrichtung für weibliche Personen umfassend einen Auffangbehälter zur Aufnahme des abzuleitenden Harns, einen sich in Abführrichtung des Harns an den Auffangbehälter anschließenden Ablauftrichter sowie einen vom Auffangbehälter wegführenden Führungssteg zur verstellbaren Aufnahme eines separat ausgebildeten Verankerungselementes, wobei das Verankerungselement in den Vaginalkanal der weiblichen Person einführbar ist.

Harnableitungs- oder Harnauffangvorrichtungen sind schon lange und in einer Vielzahl von Ausprägungen am Markt verfügbar. Neben dem sicheren Ableiten und Sammeln des austretenden Harns soll ein möglichst hoher Tragekomfort gegeben sein. Zudem sind die Harnableitvorrichtungen möglichst wieder verwendbar. Ein hoher Tragekomfort wird unter anderem auch dadurch erreicht, dass die Harnableitvorrichtung einen entsprechenden sicheren Halt beim Tragen am Körper gewährleistet. Des Weiteren soll die Harnableitvorrichtung den anatomischen Gegebenheiten der Benutzerin angepasst werden können. Die in der US 4,194,508 A1 beschriebene Harnableitvorrichtung für weibliche Personen weist hierfür einen Führungssteg zur verstellbaren Aufnahme eines separat ausgebildeten, dornartigen Verankerungselementes auf, wobei das bekannte Verankerungselement in den Vaginalkanal der weiblichen Person einführbar ist. Durch die Verstellbarkeit des Verankerungselements auf und entlang dem Führungssteg kann die bekannte Vorrichtung an die unterschiedlichen anatomischen Gegebenheiten der Benutzerinnen angepasst werden. Allerdings wird das bekannte dornartige Verankerungselement auf den Führungssteg lediglich aufgeschoben und ist dort verschiebbar gelagert. Durch die Verschiebbarkeit ist kein sicherer Halt der Vorrichtung bei Benutzung gegeben, so dass der Tragekomfort für die Benutzerin erheblich gemindert wird. Es ist daher Aufgabe der vorliegenden Erfindung, eine Harnableitvorrichtung für weibliche Personen der eingangs genannten Art bereitzustellen, welche eine einfache Größenanpassung der Vorrichtung an die anatomischen Gegebenheiten der Benutzerin und eine damit verbundene sichere Anbringung der Vorrichtung gewährleistet. Zur Lösung dieser Aufgabe dient eine gattungsgemäße Harnableitvorrichtung mit den Merkmalen des Anspruchs 1.

Vorteilhafte Ausgestaltungen der Erfindung sind in den Unteransprüchen beschrieben.

Eine erfindungsgemäße Harnableitvorrichtung für weibliche Personen umfasst einen Auffangbehälter zur Aufnahme des abzuleitenden Harns, einen sich in Abführrichtung des Harns an den Auffangbehälter anschließenden Ablauftrichter sowie einen vom Auffangbehälter wegführenden Führungssteg zur verstellbaren Aufnahme eines separat ausgebildeten Verankerungselementes, wobei das Verankerungselement in den Vaginalkanal der weiblichen Person einführbar ist. Der Führungssteg weist dabei mindestens zwei Öffnungen und/oder randlich angeordnete Ausnehmungen entlang seiner Längserstreckung zur Aufnahme und zur definierten Positionierung und Feststellung des Verankerungselementes auf. Durch die Möglichkeit der definierten Positionierung und Feststellung des Verankerungselementes sind ein fester Sitz dieses Elementes an der Harnableitvorrichtung und ein entsprechend sicherer Halt der Harnableitvorrichtung im Gebrauch gewährleistet. Durch die Ausbildung von mindestens zwei Öffnungen und/oder randlich angeordneten Ausnehmungen ist die Lage des Verankerungselementes und damit die Harnableitvorrichtung insgesamt an die anatomischen Gegebenheiten der Benutzerin anpassbar. Dabei können die Öffnungen miteinander in Verbindung stehen und mindestens eine Gesamtöffnung ausbilden. Die Gesamtöffnung kann wiederum parallel zur Längserstreckung des Führungsstegs gerade verlaufend angeordnet sein, wobei zudem ein- oder beidseitig zu dieser Gesamtöffnung mit dieser verbundene Verrastöffnungen ausgebildet sind, wobei die Verrastöffnungen entlang der Längserstreckung des Führungsstegs voneinander beabstandet angeordnet sind. Diese Verrastöffnungen können dabei ungefähr senkrecht zur Längsachse der Gesamtöffnung und des Führungsstegs verlaufen und angeordnet sein. Damit ergibt sich im und am Führungssteg ein Verrastmuster, welches die unterschiedlichen Anordnungsmöglichkeiten des Verankerungselementes entlang des Führungsstegs und damit die Lage des Vaginalkanals der Benutzerin widerspiegelt. Es ist aber auch möglich, dass die Gesamtöffnung mäandrierend, zickzackförmig oder wellenförmig ausgebildet und parallel zur Längserstreckung des Führungsstegs verlaufend angeordnet ist. Dabei kann mindestens ein Vorsprung in die Gesamtöffnung hineinragend ausgebildet ist. Durch den Vorsprung wird ein unbeabsichtigtes Verschieben des Verankerungselementes in Längsrichtung des Führungsstegs zuverlässig verhindert. Durch die Anordnung mehrerer Vorsprünge entlang der Längserstreckung des Führungsstegs kann dieser in unterschiedliche Abschnitte eingeteilt werden, die wiederum die individuelle Lage des Vaginalkanals abbilden.

In einer weiteren vorteilhaften Ausgestaltung der erfindungsgemäßen Harnableitvorrichtung besteht der Auffangbehälter aus einem in die Scheide der weiblichen Person einführbaren Aufnahmerohr mit einem in Einführrichtung angeordneten, zumindest teilweise umlaufenden Kragen und einem an dem dem Kragen gegenüberliegenden Ende des Aufnahmerohrs angeordneten Auffangbehälter. Der Kragen des Aufnahmerohrs dient dabei zur Fixierung des Auffangbehälters innerhalb der Scheide der weiblichen Person. Der Kragen kann dabei eine in Längserstreckung der Harnableitvorrichtung konkave, sattelförmige Gestaltung aufweisen. Dadurch ergibt sich wiederum eine Anpassung dieses Bereichs der Harnableitvorrichtung an die Anatomie der weiblichen Person, so dass der Tragekomfort wiederum erhöht wird. Zudem kann der Durchmesser des Aufnahmerohrs kleiner sein als der Durchmesser des Kragens, so dass eine sichere Verankerung dieses Bereichs der Harnableitvorrichtung in der Scheide der Benutzerin gewährleistet ist. In weiteren vorteilhaften Ausgestaltungen der Erfindung weist das Aufnahmerohr in der Draufsicht einen runden oder ovalen Querschnitt auf. Eine derartige Ausgestaltung erhöht wiederum den Tragekomfort. Des Weiteren kann innerhalb des Aufnahmerohrs mindestens ein, zumindest teilweise umlaufender Verstärkungsrings ausgebildet sein. Dadurch wird zuverlässig ein ungewolltes Abknicken des Aufnahmerohrs verhindert.

In einer weiteren vorteilhaften Ausgestaltung der erfindungsgemäßen Harnableitvorrichtung ist an dem Ablauftrichter mindestens ein Schlauchanschluss angeordnet. Dadurch ist ein sicheres Ableiten des austretenden Harns zu weiteren Sammelbehältern gewährleistet. Des Weiteren ist es möglich, dass der Schlauchanschluß einen flexibel ausgebildeten Schlauchabschnitt aufweist. Durch die zumindest teilweise flexible Ausgestaltung des Schlauchanschlusses wird der Tragekomfort der Vorrichtung deutlich erhöht. Zudem kann der flexible Schlauchabschnitt balgartig oder ziehharmonikaartig ausgebildet sein, so dass ein unbeabsichtigtes Abknicken dieses Bereiches des Schlauchanschlusses zuverlässig verhindert wird.

In weiteren vorteilhaften Ausgestaltungen der erfindungsgemäßen Harnableitvorrichtung weist diese im Bereich unterhalb des Auffangbehälters mindestens eine vom Auffangbehälter wegführende Befestigungslasche auf. Die Befestigungslasche kann dabei mindestens ein Befestigungsmittel zur lösbaren Befestigung an einem Bekleidungsstück, insbesondere an einer Unterhose der weiblichen Person aufweisen. Dadurch ist wiederum ein sicherer Halt bei entsprechendem Tragekomfort der Harnableitvorrichtung gewährleistet.

In weiteren vorteilhaften Ausgestaltungen der erfindungsgemäßen Harnableitvorrichtung umfasst das Verankerungselement einen zylinderförmigen Grundkörper mit einem in Einführrichtung abgerundeten Endbereich sowie eine dem Endbereich gegenüberliegende Endkappe, wobei die Endkappe mit dem Grundkörper über einen Verbindungszapfen verbunden ist und der Durchmesser oder die Kantenlänge des Verbindungszapfens kleiner ist als der Durchmesser des Grundkörpers. Dabei kann der Verbindungszapfen aus einem ersten, mit dem Grundkörper verbundenen Zapfenabschnitt und einem zweiten, mit dem ersten Zapfenelement und der Endkappe verbundenen Zapfenabschnitt bestehen. Der Durchmesser oder die Kantenlänge des Verbindungszapfens ist dabei insbesondere derart gewählt, dass er in den Öffnungen und/oder den Ausnehmungen des Führungsstegs verschiebbar und verrastbar ist. Durch eine derartige Ausgestaltung des Verankerungselementes ergibt sich ein sicherer Halt dieser am Führungssteg. Des Weiteren kann am Grundkörper mindestens eine parallel zur Längserstreckung des Verankerungselementes verlaufende Drainagerille ausgebildet sein. Damit ist gewährleistet, dass eventuell auftretende Körperflüssigkeiten aus dem Vaginalkanal austreten können, so dass es zu einem Flüssigkeitsstau in diesem Bereich kommt. Zudem kann das Verankerungselement einstückig ausgebildet sein.

In weiteren vorteilhaften Ausgestaltungen der Harnableitvorrichtung bestehen alle verwendeten Elemente aus Silikon oder einer silikonenthaltenden Verbindung. Diese ist insbesondere atoxisch und hautneutral, so dass eine mögliche Allergiegefahr, wie sie bei der Verwendung von Latexmaterialien besteht, mit großer Sicherheit ausgeschlossen werden kann. Des Weiteren können der Auffangbehälter, der Ablauftrichter, die Befestigungslasche und der Führungssteg einstückig ausgebildet sein. Es ist aber auch möglich, dass lediglich der Auffangbehälter, der Ablauftrichter und die Befestigungslasche einstückig ausgebildet sind. Der Führungssteg bildet ein separates Element, das mit dem Auffangbehälter oder dem Ablauftrichter zu verbinden ist.

Weitere Einzelheiten, Merkmale und Vorteile der Erfindung ergeben sich aus dem in den folgenden Zeichnungen dargestellten Ausführungsbeispiel. Es zeigen:
- Figur 1: eine schematisch dargestellte Draufsicht auf eine erfindungsgemäße Harnableitvorrichtung,
- Figur 2: eine schematisch dargestellte Schnittansicht der erfindungsgemäßen Harnableitvorrichtung gemäß Figur 1; und
- Figur 3: eine schematische Darstellung eines Verankerungselements der erfindungsgemäßen Harnableitvorrichtung, und
- Figur 4: eine schematisch dargestellte Draufsicht auf eine erfindungsgemäße Harnableitvorrichtung gemäß einer zweiten Ausführungsform;
- Figur 5: eine schematisch dargestellte Schnittansicht der erfindungsgemäßen Harnableitvorrichtung gemäß Figur 4; und
- Figur 6: eine schematische Darstellung eines Verankerungselements der erfindungsgemäßen Harnableitvorrichtung gemäß einer zweiten Ausführungsform.

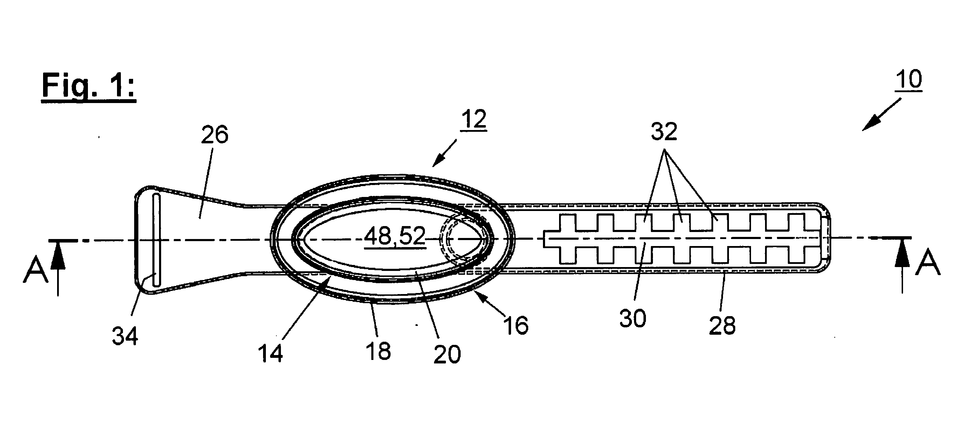

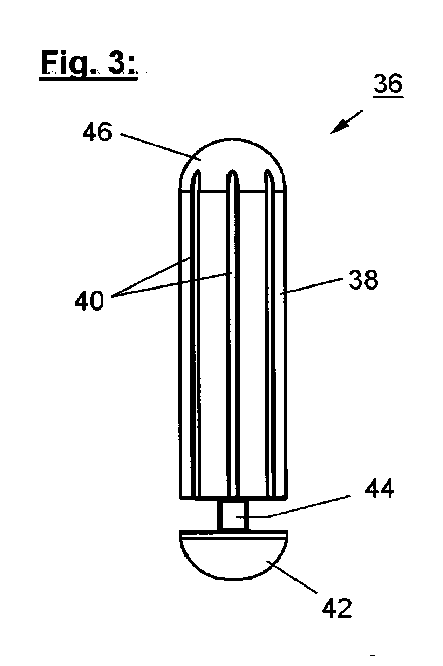

Figur 1 zeigt eine schematisch dargestellte Draufsicht auf eine Harnableitvorrichtung 10 für weibliche Personen. Die Harnableitvorrichtung 10 umfasst dabei einen Auffangbehälter 12 zur Aufnahme des abzuleitenden Harns, einen vom Auffangbehälter 12 wegführenden Führungssteg 28 zur verstellbaren Aufnahme eines separat ausgebildeten Verankerungselementes 36 (vergleiche Figur 3), wobei das Verankerungselement 36 in den Vaginalkanal der weiblichen Person einführbar ist. An dem dem Führungssteg 28 gegenüberliegenden Ende der Harnableitvorrichtung 10 ist im Bereich unterhalb des Auffangbehälters 12 eine vom Auffangbehälter 12 wegführende Befestigungslasche 26 ausgebildet. Die Befestigungslasche 26 weist in dem dargestellten Ausführungsbeispiel eine schlitzförmige Öffnung 34 auf, durch die eine Schlaufe, die wiederum an einem Bekleidungsstück, insbesondere an einer Unterhose befestigt ist, durchführbar ist. Die Befestigungslasche 26 kann aber auch andere Befestigungsmittel zur lösbaren Befestigung der Harnableitvorrichtung 10 an dem entsprechenden Bekleidungsstück der Benutzerin der Harnableitvorrichtung 10 aufweisen. Dabei sind Klett- oder Druckverschlüsse wie auch Klebefolien denkbar.

Man erkennt, dass der Führungssteg 28 mehrere Öffnungen entlang seiner Längserstreckung zur Aufnahme und zur definierten Positionierung und Feststellung des Verankerungselementes 36 aufweist. In dem dargestellten Ausführungsbeispiel stehen die Öffnungen miteinander in Verbindung und bilden eine Gesamtöffnung 30 aus. Des Weiteren erkennt man, dass die Gesamtöffnung 30 parallel zur Längserstreckung des Führungsstegs 28 gerade verlaufend angeordnet ist und beidseitig zur Gesamtöffnung 30 mit dieser verbundene Verrastöffnungen 32 ausgebildet sind. Die Verrastöffnungen 32 sind dabei einerseits entlang der Längserstreckung des Führungsstegs 28 voneinander beabstandet angeordnet und verlaufen ungefähr senkrecht zur Längsachse der Gesamtöffnung 30 und des Führungsstegs 28. Auch andere Verrastmuster sind denkbar.

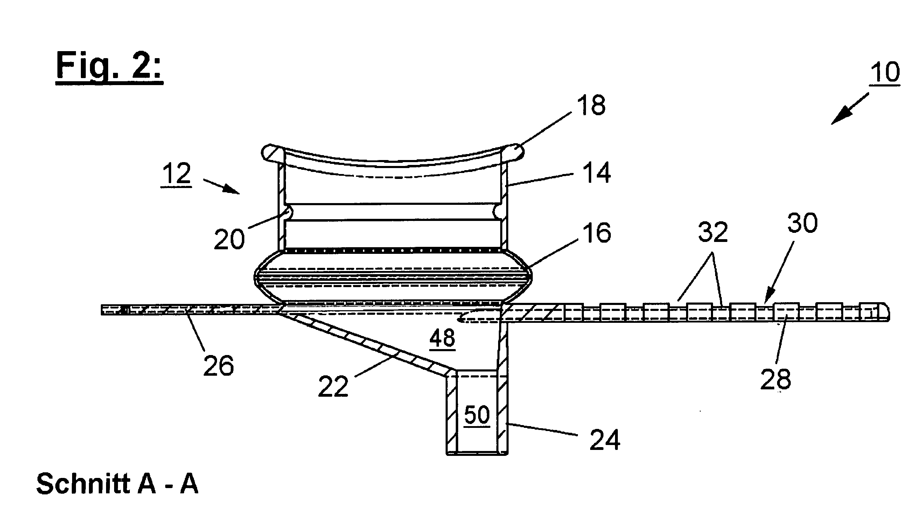

In der Draufsicht wird zudem deutlich, dass der Auffangbehälter 12 aus einem in die Scheide der weiblichen Person einführbaren Aufnahmerohr 14, einem in Einführrichtung am Aufnahmerohr 14 angeordneten umlaufenden Kragen 18 und einem an dem dem Kragen 18 gegenüberliegenden Ende des Aufnahmerohrs 14 angeordneten Auffangbehälter 16 besteht. Das Aufnahmerohr 14 weist in der Draufsicht einen ovalen Querschnitt auf. Der Durchmesser des Aufnahmerohrs 14 ist dabei kleiner als der Durchmesser des Kragens 18, der ebenfalls in der Draufsicht oval ausgebildet ist. Die Längsachsen des Aufnahmerohrs 14 und des Kragens 18 sowie des ebenfalls in der Draufsicht einen ovalen Querschnitt aufweisenden Aufnahmebehälters 16 verlaufen dabei parallel zur Längserstreckung der Harnableitvorrichtung 10 beziehungsweise der Längserstreckung des Führungsstegs 28 und der Befestigungslasche 26. Des Weiteren wird deutlich, dass der Durchmesser des Aufnahmerohrs 14 kleiner ist als der Durchmesser des Kragens 18. Damit ergibt sich eine Verankerungsmöglichkeit dieses Bereichs des Auffangbehälters 12 innerhalb der Scheide der weiblichen Person. Der Durchmesser des Auffangbehälters 16 ist wiederum größer als der Durchmesser des Kragens 18. Auch andere Ausgestaltungsmöglichkeiten des Auffangbehälters 12 und seiner im Vorhergehenden beschriebenen Einzelelemente ist denkbar. Für die Aufnahme des abzuleitenden Harns bildet der Auffangbehälter 12 einen Auffangraum 52 aus, der mit einem Aufnahmeraum 48 eines in Figur 2 dargestellten Ablauftrichters 22 flüssigkeitsleitend verbunden ist.

Figur 2 zeigt eine schematisch dargestellte Schnittansicht der Harnableitvorrichtung 10 gemäß Figur 1 (vergleiche Schnittlinie A - A). Man erkennt deutlich den Aufbau der Harnableitvorrichtung 10 bestehend aus dem Auffangbehälter 12, dem sich in Abführrichtung des Harns an den Auffangbehälter 12 anschließenden Ablauftrichter 22 sowie den vom Auffangbehälter 12 wegführenden Führungssteg 28. Man erkennt, dass innerhalb des Aufnahmerohrs 14 ein umlaufender Verstärkungsring 20 ausgebildet ist. Dieser dient zur Stabilitätsverstärkung des Aufnahmerohrs 14. Des Weiteren wird deutlich, dass der Kragen 18 eine in Längserstreckung der Harnableitvorrichtung 10 konkave, sattelförmige Gestaltung aufweist. Auch der deutlich größere Durchmesser des Kragens im Vergleich zum Aufnahmerohr 14 wird deutlich erkennbar. Der Aufnahmebehälter 16 weist in dem dargestellten Ausführungsbeispiel eine scheibenartige Form mit abgerundeten Seitenenden auf. Aber auch andere Ausgestaltungsformen sind denkbar. An dem Ablauftrichter 22 ist zudem ein Schlauchanschluss 24 angeordnet. Innerhalb des Schlauchanschlusses 24 ist ein Ableitraum 50 für den Abfluss des austretenden Harns ausgebildet, wobei der Ableitraum 50 flüssigkeitsleitend mit dem Aufnahmeraum 48 des Ablauftrichters 22 verbunden ist. In dem dargestellten Ausführungsbeispiel besteht die gesamte Harnableitvorrichtung 10 aus Silikon beziehungsweise einer Silikonmischung. Der Auffangbehälter 12, der Ablauftrichter 22, die Befestigungslasche 26 und der Führungssteg 28 sind dabei einstückig ausgebildet.

Figur 3 zeigt eine schematische Darstellung des Verankerungselementes 36 mit der Harnablaufvorrichtung 10. Das Verankerungselement 36 ist als separates Element ausgebildet und besteht in dem dargestellten Ausführungsbeispiel wiederum aus Silikon oder einer entsprechenden Silikonmischung. Das Verankerungselement 36 weist dabei einen zylinderförmigen Grundkörper 38 mit einem in Einführrichtung abgerundeten Endbereich 46 sowie eine dem Endbereich 46 gegenüberliegende Endkappe 42 auf. Die Endkappe 42 ist dabei mit dem Grundkörper 38 über einen Verbindungszapfen 44 verbunden. Man erkennt, dass der Durchmesser des Verbindungszapfens 44 kleiner ist als der Durchmesser des Grundkörpers 38. Dabei wird der Durchmesser des Verbindungszapfens 44 insbesondere derart gewählt, dass er in den Öffnungen und/oder Ausnehmungen des Führungsstegs 28 verschiebbar und verrastbar ist. In dem dargestellten Ausführungsbeispiel weist der Grundkörper 38 mehrere parallel zu seiner Längserstreckung verlaufende Drainagerillen 40 auf. Zudem ist das Verankerungselement 36 einstückig ausgebildet.

Die Harnableitvorrichtung 10 gewährleistet, dass über den Auffangbehälter 12, der am Ausgang der Harnröhre der weiblichen Person angeordnet ist, der Urin zuverlässig aufgefangen und abgeleitet wird. Über die spezielle Ausformung des Kragens 18 ist eine optimale Anpassung dieses Bereichs des Auffangbehälters 12 an die Anatomie der Frau gewährleistet. Zudem entsteht eine flüssigkeitsdichte Verbindung. Durch die beschriebene Ausgestaltung des Führungsstegs 28 mit der Möglichkeit der Positionierung und Feststellung des Verankerungselementes 36 wird die Harnableitvorrichtung 10 und insbesondere der Auffangbehälter 12 sicher an dem vorbestimmten Platz gehalten. Zudem kann die Harnableitvorrichtung 10 über die Befestigungslasche 26 an einem Bekleidungsstück, insbesondere einer Unterhose der weiblichen Person lösbar befestigt und damit sicher gehalten werden.

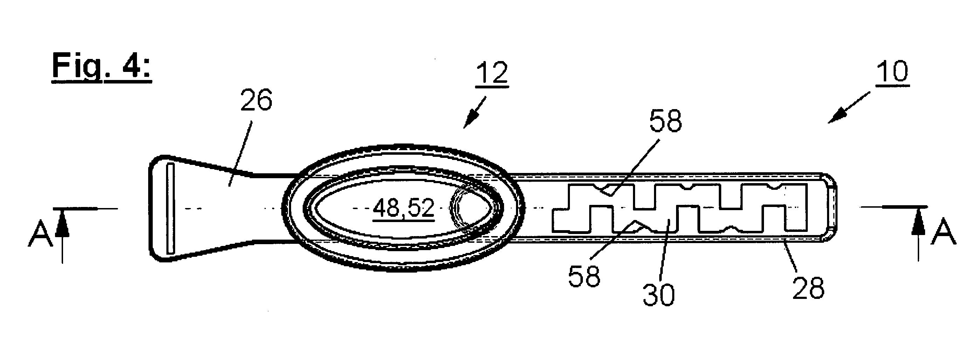

Figur 4 zeigt eine schematisch dargestellte Draufsicht auf eine Harnableitvorrichtung 10 für weibliche Personen gemäß einer zweiten Ausführungsform. Im Unterschied zu der in den Figuren 1 und 2 dargestellten Ausführungsform weist hier der Führungssteg 28 eine mäanderförmige Gesamtöffnung 30 auf. Die Gesamtöffnung 30 erstreckt sich wiederum in der Längserstreckung des Führungsstegs 28. Man erkennt, dass innerhalb der Gesamtöffnung 30 mehrere Vorsprünge 58 ausgebildet sind. Die Vorsprünge 58 ragen dabei in die Gesamtöffnung 30 hinein. Die Vorsprünge 58 verhindern ein unerwünschtes Verschieben des Verankerungselementes 36 innerhalb der Gesamtöffnung 30 des Führungsstegs 28. Die Ausgestaltung des Auffangbehälters 12 sowie der Befestigungslasche 26 entspricht der Ausgestaltung der in den Figuren 1 und 2 dargestellten Ausführungsform.

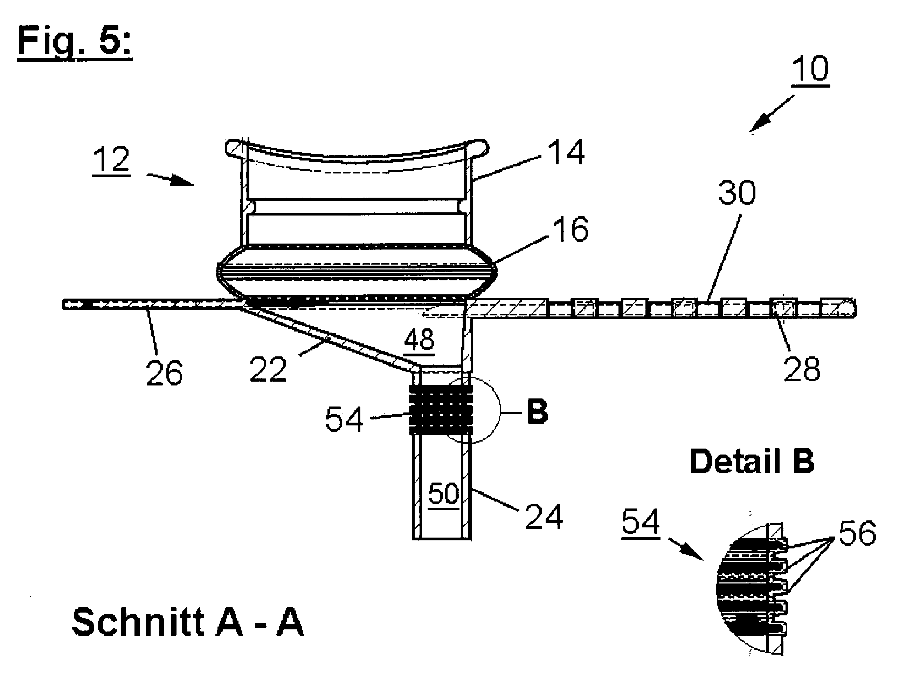

Figur 5 zeigt eine schematisch dargestellte Schnittansicht der Harnableitvorrichtung 10 gemäß Figur 4. Man erkennt, dass der Schlauchanschluß 24 des Ablauftrichters 22 einen flexiblen Schlauchabschnitt 54 aufweist. Der flexible Schlauchabschnitt 54 ist dabei ziehharmonikaartig aufgebaut. Aus der Detaildarstellung B wird dies besonders deutlich. Dabei wechseln rippenartige Elemente 56 mit dazwischen liegenden Verbindungsbereichen ab, so dass ein flexibler Schlauchbereich entsteht, der zudem in seiner Längserstreckung gedehnt werden kann. Dies wirkt einem unbeabsichtigten Abknicken des Schlauchanschlusses 24 entgegen. Bezüglich der weiteren Ausgestaltung der in Figur 5 dargestellten Harnableitvorrichtung 10 wird auf die Figurenbeschreibungen der Figuren 1, 2 und 4 verwiesen.

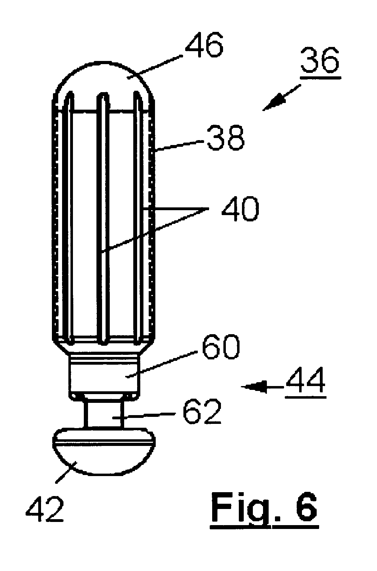

Figur 6 zeigt eine schematische Darstellung eines Verankerungselementes 36 gemäß einer zweiten Ausführungsform. Man erkennt, dass der Verbindungszapfen 44 einen ersten, mit dem Grundkörper 38 verbundenen Zapfenabschnitt 60 und einem zweiten, mit dem ersten Zapfenelement 60 und der Endkappe 42 verbundenen Zapfenabschnitt 62 besteht. Der zweite Zapfenabschnitt 62 ist dabei im Querschnitt viereckig ausgebildet. Die Kantenlänge des zweiten Zapfenabschnitts 62 ist dabei derart gewählt, dass er in der Gesamtöffnung 30 des Führungsstegs 28 verschiebbar und verrastbar ist. Wie auch bei dem in Figur 3 beschriebenen Verbindungszapfen besteht der hier beschriebene Verbindungszapfen 44 wiederum aus Silikon oder einer entsprechenden Silikonmischung. Die Kantenlänge des zweiten Zapfenabschnittes 62 kann daher geringfügig größer sein als der Durchmesser bzw. die Öffnungsweite der Gesamtöffnung 30. Durch die Verwendung der genannten flexiblen Materialien, die auch zur Herstellung des Führungsstegs 28 verwendet werden, ist mit der Wahl der genannten Durchmesser bzw. Kantenlängen einerseits ein Verschieben und andererseits eine sichere Verrastung des Verankerungselementes 36 am Führungssteg 28 möglich. Des Weiteren erkennt man, dass der Durchmesser des ersten Zapfenabschnitts 60 geringer ist als der Durchmesser des zylinderförmigen Grundkörpers 38. Der Grundkörper 38 verjüngt sich in diesem Bereich hin zum ersten Zapfenabschnitt 60, so dass ein Freiraum zwischen dem Grundkörper 38 und dem Führungssteg 28 bei eingesetztem Verankerungselement 36 entsteht. Dieser Freiraum dient zur Aufnahme und besseren Ableitung möglicher, aus dem Vaginalkanal austretender Flüssigkeiten. Die Flüssigkeiten werden dabei wiederum über mehrere am Grundkörper 38 ausgebildete und parallel zu seiner Längserstreckung verlaufende Drainagerillen 40 geleitet.

## Patentansprüche

1. Harnableitvorrichtung für weibliche Personen umfassend einen Auffangbehälter (12) zur Aufnahme des abzuleitenden Harns, einen sich in Abführrichtung des Harns an den Auffangbehälter (12) anschließenden Ablauftrichter (22) sowie einen vom Auffangbehälter (12) wegführenden Führungssteg (28) zur verstellbaren Aufnahme eines separat ausgebildeten Verankerungselementes (36), wobei das Verankerungselement (36) in den Vaginalkanal der weiblichen Person einführbar ist,
**dadurch gekennzeichnet,**
**dass** der Führungssteg (28) mindestens zwei Öffnungen und/oder randlich angeordnete Ausnehmungen entlang seiner Längserstreckung zur Aufnahme und zur definierten Positionierung und Feststellung des Verankerungselementes (36) aufweist.

2. Harnableitvorrichtung nach Anspruch 1,
**dadurch gekennzeichnet,**
**dass** die Öffnungen miteinander in Verbindung stehen und mindestens eine Gesamtöffnung (30) ausbilden.

3. Harnableitvorrichtung nach Anspruch 2,
**dadurch gekennzeichnet,**
**dass** die Gesamtöffnung (30) gerade, mäandrierend, zickzackförmig oder wellenförmig ausgebildet ist und parallel zur Längserstreckung des Führungsstegs (28) verlaufend angeordnet ist.

4. Harnableitvorrichtung nach Anspruch 3,
**dadurch gekennzeichnet,**
**dass** ein- oder beidseitig zur Gesamtöffnung (30) mit dieser verbundene Verrastöffnungen (32) ausgebildet sind, wobei die Verrastöffnungen (32) entlang der Längserstreckung des Führungsstegs (28) voneinander beabstandet angeordnet sind.

5. Harnableitvorrichtung nach Anspruch 4,
**dadurch gekennzeichnet,**
**dass** die Verrastöffnungen (32) ungefähr senkrecht zur Längsachse der Gesamtöffnung (30) und des Führungsstegs (28) verlaufen und angeordnet sind.

6. Harnableitvorrichtung nach einem der Ansprüche 2 bis 5,
**dadurch gekennzeichnet,**
**dass** mindestens ein Vorsprung (58) in die Gesamtöffnung (30) hineinragend ausgebildet ist.

7. Harnableitvorrichtung nach einem der vorhergehenden Ansprüche,
**dadurch gekennzeichnet,**
**dass** der Auffangbehälter (12) aus einem in die Scheide der weiblichen Person einführbarem Aufnahmerohr (14) mit einem in Einführrichtung angeordneten, zumindest teilweise umlaufenden Kragen (18) und einem an dem dem Kragen (18) gegenüberliegenden Ende des Aufnahmerohrs (14) angeordneten Auffangbehälter (16) besteht.

8. Harnableitvorrichtung nach Anspruch 7,
**dadurch gekennzeichnet,**
**dass** innerhalb des Aufnahmerohrs (14) mindestens ein, zumindest teilweise umlaufender Verstärkungsring (20) ausgebildet ist oder dass der Kragen (18) eine in Längserstreckung der Harnableitvorrichtung (10) konkave, sattelförmige Gestaltung aufweist.

9. Harnableitvorrichtung nach einem der Ansprüche 7 oder 8,
**dadurch gekennzeichnet,**
**dass** der Durchmesser des Aufnahmerohrs (14) kleiner ist als der Durchmesser des Kragens (18).

10. Harnableitvorrichtung nach einem der vorhergehenden Ansprüche,
**dadurch gekennzeichnet,**
**dass** die Harnableitvorrichtung (10) im Bereich unterhalb des Auffangbehälters (12) mindestens eine vom Auffangbehälter (12) wegführende Befestigungslasche (26) aufweist, wobei die Befestigungslasche (26) mindestens ein Befestigungsmittel zur lösbaren Befestigung an einem Bekleidungsstück, insbesondere an einer Unterhose der weiblichen Person aufweist.

11. Harnableitvorrichtung nach einem der vorhergehenden Ansprüche,
**dadurch gekennzeichnet,**
**dass** der Auffangbehälter (12), der Ablauftrichter (22), die Befestigungslasche (26) und der Führungssteg (28) einstückig ausgebildet sind oder dass der Auffangbehälter (12), der Ablauftrichter (22) und die Befestigungslasche (26) einstückig ausgebildet sind.

12. Harnableitvorrichtung nach einem der vorhergehenden Ansprüche,
**dadurch gekennzeichnet,**
**dass** das Verankerungselement (36) einen zylinderförmigen Grundkörper (38) mit einem in Einführrichtung abgerundetem Endbereich (46) sowie eine dem Endbereich (46) gegenüberliegende Endkappe (42) umfasst, wobei die Endkappe (42) mit dem Grundkörper (38) über einen Verbindungszapfen (44) verbunden ist und der Durchmesser oder die Kantenlänge des Verbindungszapfens (44) kleiner ist als der Durchmesser des Grundkörpers (38).

13. Harnableitvorrichtung nach Anspruch 12,
**dadurch gekennzeichnet,**
**dass** der Verbindungszapfen (44) einen ersten, mit dem Grundkörper (38) verbundenen Zapfenabschnitt (60) und einem zweiten, mit dem ersten Zapfenelement (60) und der Endkappe (42) verbundenen Zapfenabschnitt (62) besteht.

14. Harnableitvorrichtung nach Anspruch 12 oder 13,
**dadurch gekennzeichnet,**
**dass** der Durchmesser oder die Kantenlänge des Verbindungszapfens (44) oder des zweiten Zapfenabschnitts (62) derart gewählt ist, dass er in der Gesamtöffnung (30) und/oder den Öffnungen (32) und/oder den Ausnehmungen des Führungsstegs (28) verschiebbar und verrastbar ist.

15. Harnableitvorrichtung nach einem der Ansprüche 12 bis 14,
**dadurch gekennzeichnet,**
**dass** am Grundkörper (38) mindestens eine parallel zur Längserstreckung des Verankerungselements (36) verlaufende Drainagerille (40) ausgebildet ist.

## Claims

1. Urinary drain device for female persons including a collecting container (12) for receiving the urine to be drained, a drain hopper (22) adjoining the collecting container (12) in discharge direction of the urine, as well as a guiding web (28) leading away from the collecting container (12) for adjustably receiving a separately formed anchoring element (36), wherein the anchoring element (36) can be introduced into the vaginal canal of the female person,
**characterized in that**
the guiding web (28) has at least two openings and/or recesses disposed at the edge along its longitudinal extension for receiving and for defined positioning and fixing the anchoring element (36).

2. Urinary drain device according to claim 1,
**characterized in that**
the openings communicate with each other and form at least one overall opening (30).

3. Urinary drain device according to claim 2,
**characterized in that**
the overall opening (30) is formed straight, meandering, zigzag-shaped or wave-shaped and is disposed extending parallel to the longitudinal extension of the guiding web (28).

4. Urinary drain device according to claim 3,
**characterized in that**
locking openings (32) are formed one- or two-sided to the overall opening (30) connected thereto, wherein the locking openings (32) are disposed spaced from each other along the longitudinal extension of the guiding web (28).

5. Urinary drain device according to claim 4,
**characterized in that**
the locking openings (32) extend and are disposed approximately perpendicularly to the longitudinal axis of the overall opening (30) and of the guiding web (28).

6. Urinary drain device according to any one of claims 2 to 5,
**characterized in that**
at least one projection (58) is formed protruding into the overall opening (30).

7. Urinary drain device according to any one of the preceding claims,
**characterized in that**
the collecting container (12) is composed of a receiving tube (14) capable of being introduced into the vagina of the female person with an at least partially circumferential collar (18) disposed in introduction direction and a collecting container (16) disposed at the end of the receiving tube (14) opposing the collar (18).

8. Urinary drain device according to claim 7,
**characterized in that**
at least one, at least partially circumferential reinforcing ring (20) is formed within the receiving tube (14) or that the collar (18) has a saddle-shaped configuration concave in longitudinal extension of the urinary drain device (10).

9. Urinary drain device according to any one of claims 7 or 8,
**characterized in that**
the diameter of the receiving tube (14) is less than the diameter of the collar (18).

10. Urinary drain device according to any one of the preceding claims,
**characterized in that**
the urinary drain device (10) has at least one fixing flap (26) leading away from the collecting container (12) in the area below the collecting container (12), wherein the fixing flap (26) has at least one fixing means for detachably fixing to a piece of clothes, in particular to underpants of the female person.

11. Urinary drain device according to any one of the preceding claims,
**characterized in that**
the collecting container (12), the drain hopper (22), the fixing flap (26) and the guiding web (28) are integrally formed or that the collecting container (12), the drain hopper (22) and the fixing flap (26) are integrally formed.

12. Urinary drain device according to any one of the preceding claims,
**characterized in that**
the anchoring element (36) includes a cylindrical base body (38) with an end area (46) rounded in introduction direction as well as an end cap (42) opposing the end area (46), wherein the end cap (42) is connected to the base body (38) via a connecting peg (44) and the diameter or the edge length of the connecting peg (44) is less than the diameter of the base body (38).

13. Urinary drain device according to claim 12,
**characterized in that**
the connecting peg (44) is composed of a first peg section (60) connected to the base body (38) and a second peg section (62) connected to the first peg element (60) and the end cap (42).

14. Urinary drain device according to claim 12 or 13,
**characterized in that**
the diameter or the edge length of the connecting peg (44) or of the second peg section (62) is selected such that it is displaceable and lockable in the overall opening (30) and/or the openings (32) and/or the recesses of the guiding web (28).

15. Urinary drain device according to any one of claims 12 to 14,
**characterized in that**
at least one drainage groove (40) extending parallel to the longitudinal extension of the anchoring element (36) is formed on the base body (38).

## Revendications

1. Dispositif d'évacuation d'urine pour des personnes de sexe féminin, comprenant un récipient collecteur (12), destiné à recueillir l'urine à évacuer, une goulotte (22) d'écoulement, qui se raccorde, dans le sens d'évacuation de l'urine, au récipient collecteur (12), ainsi qu'une barrette (28) de guidage, partant du récipient collecteur (12), pour y loger de manière réglable un élément (36) d'ancrage constitué séparément, moyennant quoi l'élément (36) d'ancrage peut être introduit dans le canal vaginal de la personne de sexe féminin,
**caractérisé en ce**
**que** la barrette (28) de guidage présente au moins deux ouvertures et / ou des évidements agencés en périphérie le long de son extension longitudinale, destinés au logement et au positionnement défini et à la fixation de l'élément (36) d'ancrage.

2. Dispositif d'évacuation d'urine selon la revendication 1,
**caractérisé en ce**
**que** les ouvertures communiquent entre elles et constituent au moins une ouverture totale (30).

3. Dispositif d'évacuation d'urine selon la revendication 2,
**caractérisé en ce**
**que** l'ouverture totale (30) est constituée droite, méandreuse, en zigzag ou onduleuse et est agencée comme évoluant parallèlement à l'extension longitudinale de la barrette (28) de guidage.

4. Dispositif d'évacuation d'urine selon la revendication 3,
**caractérisé en ce**
**que** des ouvertures (32) d'encliquetage sont constituées d'un côté ou des deux côtés de l'ouverture totale (30) et sont reliées à celle-ci, moyennant quoi les ouvertures (32) d'encliquetage sont agencées, espacées les unes des autres, le long de l'extension longitudinale de la barrette (28) de guidage.

5. Dispositif d'évacuation d'urine selon la revendication 4,
**caractérisé en ce**
**que** les ouvertures (32) d'encliquetage évoluent et sont agencées approximativement verticalement par rapport à l'axe longitudinal de l'ouverture totale (30) et de la barrette (28) de guidage.

6. Dispositif d'évacuation d'urine selon l'une des revendications 2 à 5,
**caractérisé en ce**
**qu'**au moins une saillie (58) est constituée de manière à pénétrer dans l'ouverture totale (30).

7. Dispositif d'évacuation d'urine selon l'une des revendications précédentes,
**caractérisé en ce**
**que** le récipient collecteur (12) se compose d'un tube (14) de réception pouvant être introduit dans le vagin de la personne de sexe féminin avec un collet (18) au moins partiellement périphérique, agencé dans le sens de l'introduction et d'un récipient collecteur (12), agencé à l'extrémité du tube (14) de réception opposée au collet (18).

8. Dispositif d'évacuation d'urine selon la revendication 7,
**caractérisé en ce**
**qu'**à l'intérieur du tube (14) de réception, au moins un anneau (20) de renforcement, au moins partiellement périphérique, est constitué ou que le collet (18) présente un tracé concave, en forme de selle, dans l'extension longitudinale du dispositif (10) d'évacuation d'urine.

9. Dispositif d'évacuation d'urine selon l'une des revendications 7 ou 8,
**caractérisé en ce**
**que** le diamètre du tube (14) de réception est inférieur au diamètre du collet (18).

10. Dispositif d'évacuation d'urine selon l'une des revendications précédentes,
**caractérisé en ce**
**que** le dispositif (10) d'évacuation d'urine présente, dans la zone au-dessous du récipient collecteur (12), au moins une languette (26) de fixation partant du récipient collecteur (12), moyennant quoi la languette (26) de fixation présente au moins un moyen de fixation pour la fixation amovible à un vêtement, en particulier à un slip de la personne de sexe féminin.

11. Dispositif d'évacuation d'urine selon l'une des revendications précédentes,
**caractérisé en ce**
**que** le récipient collecteur (12), la goulotte (22) d'écoulement, la languette (26) de fixation et la barrette (28) de guidage sont constitués d'une seule pièce ou que le récipient collecteur (12), la goulotte (22) d'écoulement et la languette (26) de fixation sont constitués d'une seule pièce.

12. Dispositif d'évacuation d'urine selon l'une des revendications précédentes,
**caractérisé en ce**
**que** l'élément (36) d'ancrage comprend un corps (38) de base de forme cylindrique avec une région (46) d'extrémité arrondie dans le sens d'introduction, ainsi qu'un embout (42) opposé à la région (46) d'extrémité, moyennant quoi l'embout (42) est relié au corps (38) de base par l'intermédiaire d'un pivot (44) de liaison et le diamètre ou la longueur d'arête du pivot (44) de liaison est inférieur au diamètre du corps (38) de base.

13. Dispositif d'évacuation d'urine selon la revendication 12,
**caractérisé en ce**
**que** le pivot (44) de liaison se compose d'une première section (60) de pivot, reliée au corps (38) de base et d'une seconde section (62) de pivot, reliée au premier élément (60) de pivot et à l'embout (42).

14. Dispositif d'évacuation d'urine selon la revendication 12 ou 13,
**caractérisé en ce**
**que** le diamètre ou la longueur d'arête du pivot (44) de liaison ou de la seconde section (62) de pivot est choisi de telle sorte qu'il peut être déplacé et encliqueté dans l'ouverture totale (30) et / ou les ouvertures (32) et / ou les évidements de la barrette (28) de guidage.

15. Dispositif d'évacuation d'urine selon l'une des revendications 12 à 14,
**caractérisé en ce**
**qu'**au moins une rainure (40) de drainage, évoluant parallèlement à l'extension longitudinale de l'élément (36) d'ancrage, est constituée sur le corps (38) de base.
